# EUROPEAN PATENT SPECIFICATION

(11) **EP 2 968 651 B1**
(45) Date of publication and mention of the grant of the patent: **21.10.2020**
(21) Application number: 14712861.5
(22) Date of filing: 03.03.2014
(51) Int. Cl.: A61L 15/58, A61L 24/00, A61P 1/04, A61L 26/00

(54) **ADHESIVE MEDICAL PRODUCTS AND METHODS FOR TREATING GASTROINTESTINAL LESIONS**
MEDIZINISCHE HAFTPRODUKTE UND VERFAHREN ZUR BEHANDLUNG VON GASTROINTESTINALEN LÄSIONEN
PRODUITS MÉDICAUX ADHÉSIFS ET PROCÉDÉS POUR TRAITER DES LÉSIONS GASTRO-INTESTINALES

(30) Priority: 15.03.2013 US 201361793586 P
(43) Date of publication of application: 20.01.2016
(73) Proprietor: Cook Medical Technologies LLC, Bloomington, IN 47404 (US)
(72) Inventor: GITTARD, Shaun D., Winston-Salem, NC 27101 (US); SIGMON, John Crowder, Winston-Salem, NC 27104 (US); SURTI, Vihar C., Winston-Salem, NC 27104 (US)
(74) Representative: Mathys & Squire
(86) International application number: PCT/US2014/019893
(87) International publication number: WO 2014/149617

(56) References cited:
- WO-A1-02/064113
- WO-A1-2007/074327
- GB-A- 2 435 425
- US-A- 5 260 066
- US-A1- 2004 225 247
- US-A1- 2009 181 074
- US-A1- 2010 129 427

## Description

### BACKGROUND

### 1. Technical Field

The present invention relates to adhesive medical products for treating lesions in the gastrointestinal tract, such as lesions arising from the disorders of the gastrointestinal tract and or medical procedures that require removal of the mucosal or submucosal layers of gastrointestinal tract wall.

### 2. Background Information

There are several disorders of the gastrointestinal tract, e.g., gastrointestinal inflammation, gastrointestinal cancer, gastrointestinal infection, gastrointestinal motility dysfunction, or lesions, wounds or contusions of tissue of a portion of the gastrointestinal tract that can cause gastrointestinal lesions. In addition, there are a wide variety of medical procedures that require removal of the mucosal or submucosal layers of gastrointestinal tract wall and can also cause injury or lesions in the gastrointestinal tract. These procedures include endoscopic mucosal resection (EMR), endoscopic submucosal dissection, polypectomy, per-oral endoscopic myotomy, biopsy, and ablation (thermal, chemical, radiofrequency, and cryogenic). As with the disorders of the gastrointestinal tract, similar adverse events can occur after removal of the mucosal or submucosal layers, including bleeding and structuring.

Use of mucoadhesives and bioadhesives including various chemical derivatives of chitosan, Carbopol™ and polycarbophil has been known to open epithelial tight junctions, prevent intestinal ulceration, retain drugs in open wounds, increase ocular-surface residence, and have vaccine adjuvant activity, however, the use of mucoadhesives for the treatment of gastrointestinal lesion has not been previously proposed or documented.

Currently, the standard approach for treating gastrointestinal lesions is to use clips to fold the tissue upon itself, thus isolating the lesion from the gastrointestinal environment. However, this method has a poor success rate (10-20% failure), is expensive, and technically challenging.

As such, new or improved devices and methods for long-term localized treatment to address the issues arising from the disorders of the gastrointestinal tract and or medical procedures that require removal of the mucosal or submucosal layers of gastrointestinal tract wall are highly desirable.

WO 02/064113 discloses stable, viscous mucoadhesive aqueous compositions which are useful for the prevention and treatment of ulcerative, inflammatory, and/or erosive disorders of mucous membranes and/or the delivery of pharmaceutically active compounds to mucosal surfaces for topical treatment or transfer to the systemic circulation.

US 5260066 discloses a controlled release bandage containing therapeutic agents in a poly(vinyl alcohol) cryogel.

US 2009/0181074 A1 discloses preformed film for post-surgical protection of mucosal tissue comprising a barrier material and an adhesive material contacting the barrier material, wherein the barrier material and the adhesive material remain substantially intact for a period of time from about 48 hours to about 14 days after applying the apparatus to mucosal tissue.

### SUMMARY

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

The present invention relates to a medical product for protecting or treating a lesion in the gastrointestinal tract comprising a protective covering, wherein the protective covering comprises a mucoadhesive agent in a powder form and at least one of a superabsorbent material and a hemostatic material, and wherein the medical product upon application at and about the site of the lesion adheres to the gastrointestinal tissue and is capable of remaining at and about the site of the lesion for a minimum of 30 minutes, as defined in claim 1. The mucoadhesive agent is selected from carbomers (e.g., polyacrylic acids), polycyclic aromatic hydrocarbons (e.g., retene), carboxylic acids, polyvinylpyroolidones, polyvinylalchohols, polycarbophils, chitosan materials (e.g., poliglusam, deacetylchitin, or poly-(D)glucosamine), methylcellulose, methylethylcellulose, sodium carboxymethylcellulose, hydroxypropylcellulose, hydroxypropylmethylcellulose, or hydroxyethylcellulose, polyethylene glycol, lectins (e.g., Erythrina c. lectin, Concanavalin a. lectin, Ulex europaeus lectin, and C-Type lectin), thiamines (e.g. thiamine end capped polymer chains), chitosan-cysteine, chitosan-thiolbutylamidine, chitosan-thioglycolic acid, polyacrylic acid-cysteine, polyacrylic acid-cysteamine, carboxymethylellulose-cystein, or alginate-cysteine, mucin, guar gum, karaya gum, xanthan gum, locust bean gum, acacia gum, gellan gum, tragacanth, soluble starch, gelatin, pectin, and fimbrial protein biomolecules having an affinity for a mucosa The protective covering may also include a solid material in a form of a bandage, a patch, or a dressing. The medical product may further include a mechanical scaffold. The mechanical scaffold can include an extracellular matrix or a synthetic material. The mechanical scaffold may include an impermeable material. In certain embodiments, the medical product may also include a color indicator. The medical product may be for protecting or treating gastrointestinal lesions resulting from endoscopic submucosal dissection, endoscopic mucosal resection, polypectomy, ulcers, cancers, varices, Barrett's esophagus ablation, infection, anastomoses, fistulas or a combination thereof. Preferably, the protective covering is capable of remaining at and about the site of the lesion for a minimum of 24 hours; more preferably for a minimum of 72 hours.

The medical product can be used in a method for protecting or treating a lesion in the gastrointestinal tract. The method includes locally applying a medical product comprising a protective covering to and about a lesion site in the gastrointestinal tract, wherein the medical product upon application at and about the site of the lesion adheres to the gastrointestinal tissue and is capable of remaining at and about the site of the lesion for a minimum of 30 minutes. In the method, the applying step includes placing the medical product at and about the site of the lesion. In the method, the applying step may include inserting a syringe loaded with the medical product about the site of the lesion and applying the medical product at and about the site of the lesion. In the method, the applying step may include spraying, ejecting or spreading the medical product at and about the site of the lesion. In the method, the applying step may be through endoscopic techniques, laparoscopic techniques, or direct access. In certain embodiments, the present method may further include applying a crosslinking initiator selected from the group consisting of thermal, light, curing agent or a catalyst. The method may further include instructing a medical practitioner to apply the medical product to and about a lesion site in the gastrointestinal tract.

The medical product can also be used for closing a perforation, anastomosis, or fistula of the gastrointestinal tract, the medical product including a protecting covering, wherein upon the application of the medical product, the protective covering forms a seal over the perforation, anastomosis, or fistula.

### BRIEF DESCRIPTION OF THE DRAWINGS

Figure 1 is a photograph showing an example of a mucoadhesive covering (not claimed).
Figure 2 is a photograph showing an example of a powder form of the mucoadhesive covering on a post-mucosectomy site at time 0.
Figures 3A-D are photographs of tissue from the treated (A-B) and untreated (C-D) post-mucosectomy sites.

### DETAILED DESCRIPTION OF THE DRAWINGS AND THE PRESENTLY PREFERRED EMBODIMENTS

The present invention relates to adhesive medical products for protecting or treating lesions in the gastrointestinal tract comprising a protective covering, wherein the protective covering comprises a mucoadhesive agent in a powder form and at least one of a superabsorbent material and a hemostatic material, and wherein the medical product upon application at and about the site of the lesion adheres to the gastrointestinal tissue and is capable of remaining at and about the site of the lesion for a minimum of 30 minutes. The lesions in the gastrointsestinal tract may result from disorders of the gastrointestinal tract and/or medical procedures that require removal of the mucosal or submucosal layers of gastrointestinal tract, such as, for example, endoscopic submucosal dissection, endoscopic mucosal resection, polypectomy, ulcer, cancer, varices, Barrett's esophagus ablation, or a combination thereof.

The long-lasting adhesive medical products of the present invention include a protective covering that comprises a mucoadhesive agent in a powder form. The protective covering may further comprise a solid material, such as a bandage, a dressing, a patch or the like. The protective coating is delivered to and about a site of a lesion in the gastrointestinal tract to protect or treat the lesion from further injury or infection, slow or stop bleeding, prevent delayed bleeding, prevent delayed perforation, seal anastomotic leaks or fistulas, and/or promote healing at the exposed site.

The terms "protective coating" or "coating" or "protective covering" or "covering" encompass a powder as well as, a solid material, such as a bandage, a dressing, a patch and the like. Specifically, the terms encompass powder and solid materials (e.g., bandages, dressings, patches) for use in treating or protecting lesions in the gastrointestinal tract. The appended claims specify that the protective covering comprises a mucoadhesive agent in a powder form.

The term "lesion" refers to any tissue defect or bodily injury with disruption of the normal integrity of tissue structures and/or a pathological change in a bodily organ or tissue, and specifically, any bodily organ or tissue in the gastrointestinal tract. The term is also intended to encompass the terms "wound," "injury,""sore," "necrosis" and "ulcer." The term "sore" typically refers to any lesion of the mucous membranes. The term "ulcer" refers to a local defect, or excavation, of the surface of an organ or tissue in the gastrointestinal tract, which is produced by the sloughing of necrotic tissue. The term "necrosis" relates to dead tissue resulting from infection, injury, inflammation or infarctions. The lesion may be any lesion due to disorders of the gastrointestinal tract and or medical procedures that require removal of the mucosal or submucosal layers of gastrointestinal tract wall. For example, a lesion may be due to endoscopic submucosal dissection, endoscopic mucosal resection, polypectomy, ulcer, cancer, varices, Barrett's esophagus ablation, or a combination thereof. Specifically, upon a direct and localized delivery of the long-lasting adhesive medical product of the present invention to and about a site of a lesion, it will form a protective covering at and about the site of the lesion by at least partially or completely covering the lesion. The protective covering remains at and about the lesion site for a time sufficient to allow the site to be treated or healed (minimum of 30 minutes; preferably 24 hours; more preferably at least 48 or 72 hours; most preferably the protective covering is capable of remaining at and about the lesion site for 24-72 hours or longer; hence the term "long-lasting" refers to the time period that a protective covering of the present invention remains at and about the lesion and means anywhere from 30 minutes to 72 hours or longer). The term "protect" refers to protecting the site of the lesion from further injury or infection. The term "treat" refers to slowing or stopping bleeding at the site of the lesion, preventing delayed bleeding, preventing delayed perforation of the lesion, and/or promoting healing at the exposed site of the lesion, and/or promoting new tissue formation. The term "heal" in reference to a lesion refers to a process of repairing the gastrointestinal tissue by natural processes, as by, for example, scar formation so that following "healing" the lesion is at least reduced in size as compared to the initial size of the lesion or absent.

The medical product of the present invention includes a protective covering that includes a mucoadhesive agent.

A mucoadhesive agent may include any currently known or future developed mucoadhesive agent(s). As used herein, the terms "a mucoadhesive" or "a mucoadhesive agent" refer to an agent that adheres to a mucous membrane, which may line the wall of a body vessel or body cavity, e.g., a gastrointestinal surface (e.g., either or both of a gastrointestinal epithelia or mucosa (including submucosa) and, specifically, at and about a site of a lesion. The mucous membrane may include a moist mucous layer to which the mucoadhesive agent may adhere. Generally, mucoadhesive agents are hydrophilic macromolecules containing numerous functional groups capable of forming hydrogen bonds.

One example of a mucoadhesive agent suitable for use herein includes a macromolecule (e.g., a polymer) including repeating monomer units. Other examples of mucoadhesive agents for use in long-lasting adhesive medical products include, for example, a hydrophilic polymer, a hydrogel, a co-polymer, or a thiolated polymer. The hydrogen bond forming functional groups may include carboxyl groups, hydroxyl groups, carbonyl groups, sulphate groups, amide groups, or any other functional groups capable of forming hydrogen bonds. The mucoadhesive agents which are used according to the claimed invention, are selected from carbomers (e.g., polyacrylic acids), polycyclic aromatic hydrocarbons (e.g., retene), carboxylic acids, polyvinylpyroolidones, polyvinylalchohols, polycarbophils, chitosan materials (i.e., poliglusam, deacetylchitin, or poly-(D)glucosamine), methylcellulose, methylethylcellulose, sodium carboxymethylcellulose, hydroxypropylcellulose, hydroxypropylmethylcellulose, or hydroxyethylcellulose, polyethylene glycol, lectins (e.g., Erythrina c. lectin, Concanavalin a. lectin, Ulex europaeus lectin, and C-Type lectin), thiamines (e.g. thiamine end capped polymer chains); chitosan-cysteine, chitosan-thiolbutylamidine, chitosan-thioglycolic acid, polyacrylic acid-cysteine, polyacrylic acid-cysteamine, carboxymethylellulose-cystein, alginate-cysteine, mucin, guar gum, karaya gum, xanthan gum, locust bean gum, acacia gum, gellan gum, tragacanth, soluble starch, gelatin, or pectin. In some examples, mucoadhesive agents may include fimbrial protein biomolecules having an affinity for mucosal tissue.

A mucoadhesive agent adheres to a mucous membrane by physical and/or chemical forces including, for example, ionic bonding, covalent bonding, hydrogen bonding, Van-der-Waals bonding, or hydrophobic bonding (i.e., hydrophobic interaction).

According to the claimed invention, medical products described herein as coverings comprise a mucoadhesive agent that is in the form of a powder.

The amount of the mucoadhesive in the product to be administered to and about the lesion will depend on the type and size of the lesion, the form of the mucoadhesive used and the delivery system used to deliver the mucoadhesive composition. In one embodiment, the mucoadhesive in the powder form in an amount from a few tenths of a gram to several grams (e.g., anywhere from about 0.01 grams to about 25 grams) may be delivered and placed on and about the lesion site to provide a sufficient coverage at and about the lesion site, e.g., 10 microns to 2 mm thick. More typically, about 2 grams of the mucoadhesive powder would be sufficient to coat the lesion site (to achieve a thickness of anywhere from 10 microns to 2 mm thick).

In certain embodiments, any adhesive medical product or formulation described herein comprises greater than about 0.1% w/w, greater than about 0.2% w/w, greater than about 0.5% w/w, greater than about 1% w/w, greater than about 2% w/w, greater than about 3% w/w, greater than about 4% w/w, greater than about 5% w/w, greater than about 6% w/w, greater than about 7% w/w, greater than about 8% w/w, greater than about 9% w/w, greater than about 10% w/w, greater than about 11% w/w, greater than about 12% w/w, greater than about 13% w/w, greater than about 14% w/w, greater than about 15% w/w, greater than about 16% w/w, greater than about 17% w/w, greater than about 18% w/w, greater than about 19% w/w, greater than about 20% w/w, greater than about 21% w/w, greater than about 22% w/w, greater than about 23% w/w, greater than about 24% w/w, greater than about 25% w/w, greater than about 26% w/w, greater than about 27% w/w, greater than about 28% w/w, greater than about 29% w/w, greater than about 30% w/w, greater than about 35% w/w, greater than about 40% w/w, greater than about 45% w/w, greater than about 50% w/w, greater than about 55% w/w, greater than about 60% w/w, greater than about 65% w/w, greater than about 70% w/w, greater than about 80% w/w, greater than about 85% w/w, greater than about 90% w/w, greater than about 95% w/w, or greater than about 99.5% w/w of mucoadhesive that can extend the time the adhesive medical product is in contact with a surface of the gastrointestinal tract (e.g., the surface of the stomach).

As provided herein, medical products in a form of a powder may include finely divided or subdivided preparations, coarsely comminuted products, or products of intermediate particle size. A medical product in a form of a powder can comprise particles which are very coarse, of the dimensions of about 10,000 microns or 10 mm, or particles which are extremely fine, approaching dimensions of about 1 micron or less, or particles of any size in between coarse and fine. The size of the particles will depend on the type of the mucoadhesive used in the formulation and may be highly variable especially for the granular mucoadhesive.

In certain embodiments, the medical products may include more than one mucoadhesive agents. For example, a medical product in a form of a powder can be a physical admixture of two or more powdered pure mucoadhesive agents present in definite or differing proportions.

In some embodiments, therapeutic agents and or the excipients may be mixed in with the mucoadhesive powder into a powder protective covering formulation suitable for use as described herein.

Powders used for the purpose of formulations provided herein include formulations where a physician can use the formulation as is (i.e., in a powder form) followed by localized administration at and about the site of the lesion and is capable of remaining at and about the site of the lesion anywhere from 30 minutes (sufficient to stop bleeding) to 72 hours or more (sufficient to allow the tissue to heal almost completely) following the delivery.

In certain embodiments, the protective covering may include multiple materials.

The protective covering includes a mucoadhesive material (to adhere to the tissue) and at least one of a super-absorbent material (to act as a barrier to prevent liquids from contacting the lesion or blood from leaving the lesion site) and a hemostatic material (to coagulate blood in the event that the lesion bleeds).

In yet another embodiment, an additive(s) to promote healing may be included in the protective covering.

Some examples of super-absorbent materials include absorbent clays (e.g., Laponite, Smectites, Zeolites), Diatomaceous Earth, and super-absorbent polymers (e.g., sodium polyacrylate, polyacrylamide copolymer, ethylene maleic anhydride copolymer, cross-linked carboxymethylcellulose, polyvinyl alcohol copolymers, cross-linked polyethylene oxide, and starch-acrylonitrile co-polymer).

The hemostatic material used according to the claimed invention is selected from chitin, Collagen, Fibrin, Kaolinite clays, Plant-based polysaccharides, Platelets, and smectite clays.

Some examples of additives to promote healing include Aloe vera and derivatives, Honey and derivatives (i.e. Leptospermum scoparium honey), and Growth factors.

In certain embodiments, the medical product of the present invention may also include a mechanical scaffold to form a bandage, a patch, a dressing or the like. The term "scaffold" refers to any natural (e.g., extracellular matrix material or "ECM"), synthetic (e.g., woven or non-woven) material (e.g., Dacron™, electrospun materials and expanded PTFE) capable of providing a mechanical and structural support and strength to the protective covering of the adhesive medical product of the present invention.

The medical products of the present invention employ scaffolds which may be applied in a variety of forms, including single- or multi-layer sheet or mesh constructs, and/or combinations thereof.

Examples of synthetic scaffolds include biocompatible materials, such as polyesters, such as polyethylene; poly(ethylene terephthalate); fluorinated polymers, such as polytetrafluoroethylene (PTFE) and fibers of expanded PTFE; polyurethanes; silicone, etc. One example of biocompatible polyester includes Dacron™ (DuPONT, Wilmington, Del.).

Examples of natural scaffold materials include bioremodelable ECM-based materials, such as naturally-derived collagenous ECM materials isolated from suitable animal or human tissue sources. As used herein, it is within the definition of a "naturally-derived ECM" to clean, delaminate, and/or comminute the ECM, or to cross-link the collagen or other components within the ECM. It is also within the definition of naturally occurring ECM to fully or partially remove one or more components or subcomponents of the naturally occurring matrix. Bioremodelable ECM materials possess biotropic properties capable of inducing tissue remodeling. Suitable ECM materials which can be processed to provide scaffold materials include, for example, submucosal (including for example small intestinal submucosa (SIS), stomach submucosa, urinary bladder submucosa, or uterine submucosa, each of these isolated from juvenile or adult animals), renal capsule membrane, dermal collagen, amnion, dura mater, pericardium, serosa, peritoneum or basement membrane layers or materials, including liver basement membrane or epithelial basement membrane materials, and others.

An exemplary ECM sheet material is a sheet of submucosa tissue graft material (OASIS™ Wound Matrix, Cook Biotech Incorporated, West Lafayette, Ind., USA).

A medical product containing a scaffold and a mucoadhesive can be used in the treatment of a lesion in the gastrointestinal lesion. For these purposes, the scaffold material can be processed into the form of a sheet, bandage or other shape to occlude or cover at least partially the lesion site in the gastrointestinal tract.

In certain embodiments, the adhesive medical product of the present invention can be a composite medical product including additional layers such as biocompatible substrate films or layers. For example, the medical products may include a top sheet or impermeable layer to restrict passage of liquid, such as gastric acid back towards the lesion. Alternatively, or in addition, the medical product of the present invention may include an additional backing layer providing further barrier or structural support.

In certain embodiments, the adhesive may be incorporated into the scaffold by mixing, coating, spraying, impregnating the scaffold with the adhesive or may be provided as a separate adhesive layer forming an adhesive-coated margin.

In certain embodiments, the medical product of the present invention may include a mucoadhesive covering on the interface that is placed on the gastrointestinal tissue and an additional material such as a woven mesh scaffold on the lumen interface to provide improved mechanical strength to the medical product.

In certain embodiments, the protective covering of the long-lasting adhesive medical product disclosed herein and used herein may comprise excipients. Specifically, additional excipients useful herein include, by way of non-limiting example, solvents, binders, fillers, lubricants, suspension agents, flavoring agents, color indicators (e.g., dyes), sweeteners, preservatives, antioxidants, buffering agents, humectants, chelating agents, surfactants, disintegrating agents, and the like. These excipients can extend the time the adhesive medical product of the present invention is in contact with a lesion site in the gastrointestinal tract and/or can increase the interaction of the adhesive medical product with a gastrointestinal surface, such as viscosity enhancing agents or absorption enhancing agents may be used.

Optionally, the present protective covering may include one or more binder, optionally one or more filler, optionally one or more lubricant, optionally one or more suspension agent, optionally one or more flavoring agent, optionally one or more coloring agent, optionally one or more sweetener, optionally one or more preservative, optionally one or more antioxidant, optionally one or more buffering agent, optionally one or more humectant, optionally one or more chelating agent, optionally one or more disintegrating agent, and optionally one or more surfactant.

In addition, the protective covering of the present invention may include, for example, a color indicator, such as a dye that upon the application to and about the lesion site changes color. Water soluble dyes are preferable. Exemplary dyes include indigo carmine, methylene blue, fluorescent proteins, Rose Bengal, India ink, and others.

Preservatives include, for example, benzalkonium chloride, cetrimide (cetyltrimethylammonium bromide), benzoic acid, benzyl alcohol, methyl-, ethyl-, propyl- and butyl-esters of para-hydroxybenzoic acid, chlorhexidine, chlorobutanol, phenylmercuric acetate, borate and nitrate, potassium sorbate, sodium benzoate, sorbic acid, thiomersal (mercurithiosalicylate), combinations thereof, or the like.

Antioxidants include, for example, ascorbyl palmitate, butylated hydroxyanisole, butylated hydroxytoluene, monothioglycerol, sodium ascorbate, sodium formaldehyde sulfoxylate, sodium metabisulfite, BHT, BHA, sodium bisulfite, vitamin E or a derivative thereof, propyl gallate, edetate (EDTA) (e.g., disodium edetate), Diethylenetriaminepentaacetic acid (DTPA), Triglycollamate (NT), combinations thereof, or the like.

Additionally, in certain embodiments, buffering agents, humectants, or chelating agents may also be incorporated into the protective covering of the adhesive medical products of the present invention.

Exemplary buffering agents include citrate buffers (i.e., citric acid and citrate), phosphate buffers, acetate buffers, carbonate buffers (e.g., calcium carbonate, sodium bicarbonate, or the like), hydroxide (e.g., magnesium hydroxide, sodium hydroxide, or the like), combinations thereof, or the like.

Humectants include, for example, glycerine, propylene glycol, ethylene glycol, glyceryl triacetate, polyols (e.g., sorbitol, xylitol, maltitol, polydextrose), and the like.

Chelating agents include, for example, edetate (EDTA) (e.g., disodium edetate), Diethylenetriaminepentaacetic acid (DTPA), Triglycollamate (NT), or the like.

In some embodiments, the adhesive medical products described herein are pharmaceutical medical products further including at least one therapeutic agent. Exemplary therapeutic agents that may be incorporated into the long-lasting adhesive medical products of the present invention include antibiotics, antiseptic agents, proton pump inhibitors, or tissue growth promoting compounds.

Other therapeutic agents may also be included and would be known to a skilled artisan.

In certain embodiments, the adhesive medical products of the present invention are prepared for a local and direct delivery at and about a site of a lesion, where the medical product upon the delivery or application to the lesion forms a coating or a patch or the like at and about the site of the lesion and is capable of remaining at and about the site of the lesion for a time sufficient to allow the site to be treated or healed. Preferably, at least partial coverage of the lesion site is achieved; most preferably a complete coverage of the lesion site is achieved. The time sufficient to allow the site to be treated or healed may vary depending on the location, type and size of the lesion and may be anywhere from 30 minutes (sufficient to stop bleeding) to 72 hours or more (sufficient to allow the tissue to heal almost completely). Preferably, the time sufficient to allow the site to be treated or healed is at least 1 hour; more preferably the time sufficient to allow the site to be treated or healed is at least 3 hours; more preferably the time sufficient to allow the site to be treated or healed is at least 6 hours; more preferably the time sufficient to allow the site to be treated or healed is at least 10 hours; more preferably the time sufficient to allow the site to be treated or healed is at least 12 hours; more preferably the time sufficient to allow the site to be treated or healed is at least 18 hours; more preferably the time sufficient to allow the site to be treated or healed is at least 24 hours; more preferably the time sufficient to allow the site to be treated or healed is at least 36 hours; more preferably the time sufficient to allow the site to be treated or healed is at least 48 hours; more preferably the time sufficient to allow the site to be treated or healed is at least 72 hours; most preferably the time sufficient to allow the site to be treated or healed is 48-72 hours.

### DELIVERY

The adhesive medical products of the present invention may be applied or delivered to and about the site of the lesion through endoscopic techniques, laparoscopic techniques, or through direct access (i.e., surgically) using, for example any suitable catheter delivery system.

The adhesive medical products of the present invention may be delivered via an intraluminal delivery system. The adhesive medical products of the present invention may be applied via spraying, ejecting, injecting or spreading.

Where the adhesive medical product of the present invention includes a protective covering that includes multiple mucoadhesive agents, the delivery of the multiple mucoadhesive agents may be through a multi-lumen catheter. The multiple mucoadhesive agents delivered to and about the lesion site may be mixed following the delivery at and about to lesion site.

Specifically, the delivery system may include a delivery device that is sized and configured to deliver and apply the adhesive medical product of the present invention directly at a targeted tissue region within a body lumen or hollow body organ, i.e., such as the site of the lesion in the gastrointestinal tract.

Also, the delivery device can be sized and configured to accommodate passage over a guide wire. In this way, the device can be introduced over the guide wire under direct visualization from an endoscope. Specifically, the guide wire can run next to the endoscope and therefore leaves a working channel of the endoscope free. In an alternative embodiment, the delivery device can be sized and configured to be back-loaded through the working channel of the endoscope. The working channel of the endoscope thereby serves to guide the delivery device while providing direct visualization.

### USE

The medical products of the invention can find wide use in the field of medicine, and in this regard, can be adapted to provide a variety of devices and objects suitable for application to and/or implantation within a patient, and especially in the gastrointestinal tract. Disclosed herein are various methods for using these materials, for example, to replace, augment, repair, and/or otherwise suitably treat diseased or otherwise damaged or defective gastrointestinal tissue of a patient.

Illustratively, medical products of the invention can be configured as medical products suitable for healing tissue, providing hemostasis, and/or providing occlusion within the body of a patient (e.g., bandage, dressing, patch, etc.).

In some embodiments, the adhesive medical products of the present invention are configured as single- or multilayered patches or other sheet or sheet-like devices for providing support to patient tissue or otherwise treating patient's gastrointestinal tissue.

Specifically, the long-lasting adhesive medical products of the present invention may be used to protect, treat or heal a lesion site in the gastrointestinal tract. Specifically, the present adhesive medical products may be used to treat a lesion arising from the disorders of the gastrointestinal tract and or medical procedures that require removal of the mucosal or submucosal layers of gastrointestinal tract wall, such as endoscopic submucosal dissection, endoscopic mucosal resection, polypectomy, ulcer, cancer, varices, Barrett's esophagus ablation, a combination thereof, or others.

Illustratively, medical products of the present invention can be processed into various shapes and configurations, for example, a sheet form, which may be used as a bandage, patch, coating, etc.

The medical product of the present invention may be used for closing a perforation, anastomosis, or fistula of the gastrointestinal tract. The medical product comprises a protecting covering as discussed above, wherein upon the application of the medical product, the protective covering forms a seal over the perforation, anastomosis, or fistula. The medical product may be used in combination with other medical products, such as clips or sutures.

### METHODS

Also disclosed herein (but not claimed) is a method for protecting or treating a lesion in the gastrointestinal tract arising from the disorders of the gastrointestinal tract and or medical procedures that require removal of the mucosal or submucosal layers of gastrointestinal tract wall. The lesion may be a post-mucosectomy lesion.

The method includes locally applying an adhesive medical product that includes a protective covering to and about the lesion in the gastrointestinal tract. The phrases "to and about" or "at and about" in connection with the delivery of the long-lasting adhesive medical products of the present invention mean that the adhesive medical product is placed on the lesion itself as well as just immediately around the lesion to ensure as most complete coverage of the lesion as possible. Upon the application of the medical product, the product forms a protective coating or covering at the site of the lesion, where the coating is capable of remaining at and about the site of the lesion for at least 30 minutes, more preferably 24-72 hours or longer.

The composition may be applied in a powder form. The types of mucoadhesive medical products and formulations suitable for use in the method were described in detail above.

The step of applying the adhesive medical product may comprise inserting a syringe loaded with the protective covering about the site of the lesion and applying the covering at and about the site of the lesion by spraying, injecting, ejecting or spreading the composition directly at and about the site of the lesion so as to provide at least partial but more preferably a complete coverage of the lesion. As previously discussed in connection with the delivery methods, the applying may be through endoscopic, laparoscopic techniques or direct access (i.e., surgically) using a catheter-based delivery system (single lumen or multi-lumen catheter system).

Without being bound by the theory, upon the application of the adhesive medical product or protective covering of the present invention to the lesion site, the covering will remain at and about the site of the lesion for the time sufficient for the lesion to heal (anywhere from 30 minutes to 72 hours or longer). Once the lesion is healed, the covering will either get washed off or erode over time; the covering will then be passed through the digestive system for removal from the body. Alternatively, the covering will remain at the site of the lesion until the outer most mucosal layer sloughs off of sheds during a normal biological process over 2-3 weeks and the coating or covering comes off with the mucosal layer.

### EXAMPLES

### Example 1 (reference Example): Liquid Mucoadhesive Benchtop Testing

Various solutions of Carbopol™ were tested: (1) Carbopol™ dissolved in ethyl acetate, (2) Carbopol™ dissolved in ethyl alcohol, and (3) Carbopol™ dissolved in water. The Carbopol™ solutions were applied to excised stomach and intestinal tissue.

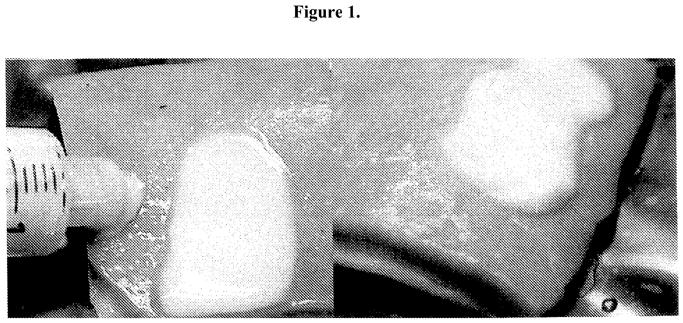

Specifically, all three solutions of Carbopol™ were loaded into a syringe and injected directly at the lesion site through a catheter. Ethyl acetate (as shown in Figure 1) was found to be a most suitable solvent to allow the highest concentration of Carbopol™. The next best was ethyl alcohol followed by water which was necessary to be highly dilute.

Next, the dye indigo carmine was mixed with the ethyl alcohol solvent. This solution remained white, with small blue particles of the dye suspended in it. However, upon application to the tissue the suspension turned blue as water from the tissue became absorbed into the adhesive. This suggests that use of a dye may be suitable for visualization of the lesion site that is being treated.

### Example 2: Animal Survival Testing

To determine the sustainability and protective effects of a coating over an extended period of time (up to 72 hours), a mucoadhesive coating was applied to post-mucosectomy sites and compared to untreated (negative control) mucosectomy sites.

Specifically, to test the sustainability of Carbopol™ 71G NF powder, the powder was sprayed onto 5 post-mucosectomy sites in a live animal and compared to five negative controls (mucosectomy sites without the application of the spray) at 72 hours post treatment. At 72 hours following the application of the Carbopol™ 71G NG powder, the animal was sacrificed and tissue samples were harvested for gross and histological examinations.

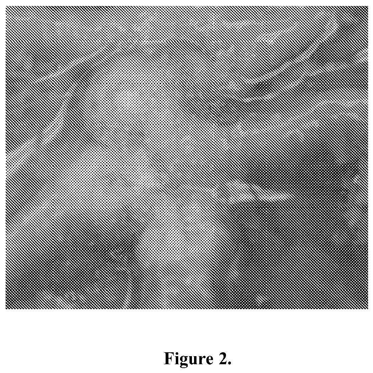

Figure 2 is an example of a powder form of the mucoadhesive coating on a post-mucosectomy site at time 0.

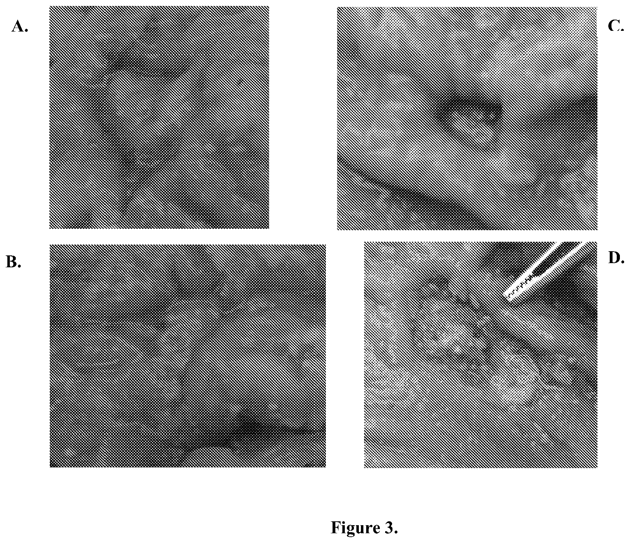

Referring to Figures 3A-D, an initial gross examination revealed a thin gel layer still residing over the Carbopol™ powder-treated post-mucosectomy sites at 72 hours following the application of the Carbopol™ powder (Figure 3A-B). Also, the Carbopol™ powder-treated post-mucosectomy sites (Figures 3A-B) looked significantly more healed as compared to the negative controls (Figures 3C-D) sites.

These results suggest that the application of a mucoadhesive coating to a post-mucosectomy site may have a protective effect and enhance healing of the injured site.

## Claims

1. A medical product for protecting or treating a lesion in the gastrointestinal tract comprising a protective covering,
wherein the protective covering comprises a mucoadhesive agent in a powder form and at least one of a superabsorbent material and a hemostatic material,
wherein the mucoadhesive agent is selected from the group consisting of carbomers, polycyclic aromatic hydrocarbons, carboxylic acids, chitosan materials, polyvinylpyrolidones, polyvinylalchohols, polycarbophils, methylcellulose, methylethylcellulose, sodium carboxymethylcellulose, hydroxypropylcellulose, hydroxypropylmethylcellulose, hydroxyethylcellulose, polyethylene glycol, lectins, thiamines, chitosan-cysteine, chitosan-thiolbutylamidine, chitosan-thioglycolic acid, polyacrylic acid-cysteine, polyacrylic acid-cysteamine, carboxymethylcellulose-cystein, alginate cysteine, mucin, guar gum, karaya gum, xanthan gum, locust bean gum, acacia gum, gellan gum, tragacanth, soluble starch, gelatin, pectin, and fimbrial protein biomolecules having an affinity for a mucosa;
wherein the hemostatic material is selected from the group consisting of chitin, collagen, fibrin, kaolinite clays, plant-based polysaccharides, platelets, and smectite clays; and
wherein the medical product upon application at and about the site of the lesion adheres to the gastrointestinal tissue and is capable of remaining at and about the site of the lesion for a minimum of 30 minutes.

2. The medical product of claim 1, wherein the protective covering further comprises a solid material in the form of a bandage, a patch or a dressing.

3. The medical product of claim 1 or claim 2, further comprising a mechanical scaffold, wherein the mechanical scaffold comprises an extracellular matrix, a synthetic material, or an impermeable material.

4. The medical product of any of claims 1-3, further comprising a color indicator.

5. The medical product of any of claims 1-4, wherein the product is suitable for protecting or treating gastrointestinal lesions resulting from endoscopic submucosal dissection, endoscopic mucosal resection, polypectomy, ulcers, cancers, varices, Barrett's esophagus ablation, infection, anastomoses, fistulas or a combination thereof.

6. The medical product of any of claims 1-5, wherein the medical product is suitable for use in closing a perforation, anastomosis, or fistula of the gastrointestinal tract.

7. The medical product of any of claims 1-6, wherein the medical product is capable of being delivered to the site of the lesion using a catheter delivery system.

8. The medical product of any of claims 1-7, wherein the medical product comprises greater than 10% w/w of the mucoadhesive agent.

9. The medical product of any of claims 1-8 for use in the treatment of a gastrointestinal lesion.

10. The medical product for use of claim 9, wherein the gastrointestinal lesions result from endoscopic submucosal dissection, endoscopic mucosal resection, polypectomy, ulcers, cancers, varices, Barrett's esophagus ablation, infection, anastomoses, fistulas or a combination thereof.

11. The medical product for use of claim 9 or claim 10, wherein the treatment comprises applying or delivering the medical product at or about the site of the lesion through endoscopic techniques, laparoscopic techniques, or surgically.

12. The medical product for use of claim 11, wherein the treatment comprises delivering the medical product using a catheter delivery system.

13. The medical product for use of any of claims 9-12, wherein the treatment comprises spraying, ejecting or spreading the medical product at and about the site of the lesion.

14. The medical product for use of any of claims 9-13, wherein the treatment comprises delivering the medical product via an intraluminal delivery system.

## Patentansprüche

1. Medizinprodukt zum Schützen oder Behandeln einer Läsion in dem Gastrointestinaltrakt, umfassend eine Schutzabdeckung,
wobei die Schutzabdeckung ein mukoadhäsives Mittel in einer Pulverform und wenigstens eines von einem superabsorbierenden Material und einem hämostatischen Material umfasst,
wobei das mukoadhäsive Mittel ausgewählt ist aus der Gruppe bestehend aus Carbomeren, polycyclischen aromatischen Kohlenwasserstoffen, Carbonsäuren, Chitosanmaterialien, Polyvinylpyrrolidonen, Polyvinylalkoholen, Polycarbophilen, Methylcellulose, Methylethylcellulose, Natriumcarboxymethylcellulose, Hydroxypropylcellulose, Hydroxypropylmethylcellulose, Hydroxyethylcellulose, Polyethylenglycol, Lectinen, Thiaminen, Chitosan-Cystein, Chitosan-Thiolbutylamidin, Chitosan-Thioglycolsäure, Polyacrylsäure-Cystein, Polyacrylsäure-Cysteamin, Carboxymethylcellulose-Cystein, Alginat-Cystein, Mucin, Guargummi, Karayagummi, Xanthangummi, Johannisbrotkernmehl, Akaziengummi, Gellangummi, Tragant, löslicher Stärke, Gelatine, Pektin und Fimbrienprotein-Biomolekülen mit einer Affinität für eine Schleimhaut;
wobei das hämostatische Material ausgewählt ist aus der Gruppe betsehend aus Chitin, Collagen, Fibrin, Kaolinittonen, Polysacchariden auf Pflanzenbasis, Plättchen und Smektittonen; und
wobei das Medizinprodukt nach Aufbringen an und um die Stelle der Läsion an dem Gastrointestinalgewebe haftet und fähig ist, für wenigstens 30 Minuten an und um die Stelle der Läsion zu verbleiben.

2. Medizinprodukt gemäß Anspruch 1, wobei die Schutzabdeckung ferner ein festes Material in der Form einer Bandage, eines Pflasters oder eines Verbands umfasst.

3. Medizinprodukt gemäß Anspruch 1 oder Anspruch 2, ferner umfassend ein mechanisches Gerüst, wobei das mechanische Gerüst eine extrazelluläre Matrix, ein synthetisches Material oder ein undurchlässiges Material umfasst.

4. Medizinprodukt gemäß einem der Ansprüche 1-3, ferner umfassend einen Farbindikator.

5. Medizinprodukt gemäß einem der Ansprüche 1-4, wobei das Produkt zum Schützen oder Behandeln von gastrointestoinalen Läsionen als Folge von endoskopischer Submukosadissektion, endoskopischer Mukosaresektion, Polypektomie, Geschwüren, Krebs, Varizen, Barrett-Ösophagusablation, Infektion, Anastomosen, Fisteln oder eine Kombination davon geeignet ist.

6. Medizinprodukt gemäß einem der Ansprüche 1-5, wobei das Medizinprodukt zur Verwendung zum Schließen einer Perforation, Anastomose oder Fistel des Gastrointestinaltrakts geeignet ist.

7. Medizinprodukt gemäß einem der Ansprüche 1-6, wobei das Medizinprodukt geeignet ist, unter Verwendung eines Katheterabgabesystems an die Stelle der Läsion abgegeben zu werden.

8. Medizinprodukt gemäß einem der Ansprüche 1-7, wobei das Medizinprodukt mehr als 10 % Gew./Gew. an dem mukoadhäsiven Mittel umfasst.

9. Medizinprodukt gemäß einem der Ansprüche 1-8 zur Verwendung bei der Behandlung einer gastrointestinalen Läsion.

10. Medizinprodukt zur Verwendung gemäß Anspruch 9, wobei die gastrointestinalen Läsionen aus endoskopischer Submukosadissektion, endoskopischer Mukosaresektion, Polypektomie, Geschwüren, Krebs, Varizen, Barrett-Ösophagusablation, Infektion, Anastomosen, Fisteln oder eine Kombination davon folgen.

11. Medizinprodukt zur Verwendung gemäß Anspruch 9 oder Anspruch 10, wobei die Behandlung Aufbringen oder Abgeben des Medizinprodukts an oder um die Stelle der Läsion durch endoskopische Verfahren, laparoskopische Verfahren oder chirurgisch umfasst.

12. Medizinprodukt zur Verwendung gemäß Anspruch 11, wobei die Behandlung Abgeben des Medizinprodukts unter Verwendung eines Katheterabgabesystems umfasst.

13. Medizinprodukt zur Verwendung gemäß einem der Ansprüche 9-12, wobei die Behandlung Sprühen, Spritzen oder Streichen des Medizinprodukts an und um die Stelle der Läsion umfasst.

14. Medizinprodukt zur Verwendung gemäß einem der Ansprüche 9-13, wobei die Behandlung Abgeben des Medizinprodukts über ein intraluminales Abgabesystem umfasst.

## Revendications

1. Produit médical pour la protection ou le traitement d'une lésion du tractus gastro-intestinal comprenant un revêtement protecteur,
dans lequel le revêtement protecteur comprend un agent muco-adhésif sous forme de poudre et au moins l'un parmi un matériau superabsorbant et un matériau hémostatique,
dans lequel l'agent muco-adhésif est choisi dans le groupe constitué par les carbomères, les hydrocarbures aromatiques polycycliques, les acides carboxyliques, les matériaux de type chitosane, les polyvinylpyrolidones, les alcools polyvinyliques, les polycarbophiles, la méthylcellulose, la méthyléthylcellulose, la carboxyméthylcellulose sodique, l'hydroxypropylcellulose, l'hydroxypropylméthylcellulose, l'hydroxyéthylcellulose, le polyéthylène glycol, les lectines, les thiamines, la chitosane-cystéine, la chitosane-thiolbutylamidine, l'acide chitosane-thioglycolique, l'acide polyacrylique-cystéine, l'acide polyacrylique-cystéamine, la carboxyméthylcellulose-cystéine, l'alginate-cysteine, la mucine, la gomme de guar, la gomme karaya, la gomme xanthane, la gomme de caroube, la gomme d'acacia, la gomme gellane, la gomme adragante, l'amidon soluble, la gélatine, la pectine, et des biomolécules de protéines de fimbriae ayant une affinité pour une muqueuse ;
dans lequel le matériau hémostatique est choisi dans le groupe constitué par la chitine, le collagène, la fibrine, les argiles de kaolinite, les polysaccharides à base de plantes, les plaquettes et les argiles de smectite ; et
dans lequel, lors de l'application au niveau et autour du site de la lésion, le produit médical adhère au tissu gastro-intestinal et est capable de rester au niveau et autour du site de la lésion pendant un minimum de 30 minutes.

2. Produit médical selon la revendication 1, dans lequel le revêtement protecteur comprend en outre un matériau solide sous la forme d'un bandage, d'un timbre ou d'un pansement.

3. Produit médical selon la revendication 1 ou la revendication 2, comprenant en outre un échafaudage mécanique, dans lequel l'échafaudage mécanique comprend une matrice extracellulaire, un matériau synthétique ou un matériau imperméable.

4. Produit médical selon l'une quelconque des revendications 1 à 3, comprenant en outre un indicateur coloré.

5. Produit médical selon l'une quelconque des revendications 1 à 4, dans lequel le produit est adapté à la protection ou au traitement de lésions gastro-intestinales résultant d'une dissection sous-muqueuse endoscopique, d'une résection muqueuse endoscopique, d'une polypectomie, d'ulcères, de cancers, de varices, de l'ablation de l'endobrachyœsophage, d'une infection, d'anastomoses, de fistules ou d'une combinaison de ceux-ci.

6. Produit médical selon l'une quelconque des revendications 1 à 5, dans lequel le produit médical est adapté à une utilisation dans la fermeture d'une perforation, d'une anastomose ou d'une fistule du tractus gastro-intestinal.

7. Produit médical selon l'une quelconque des revendications 1 à 6, dans lequel le produit médical est capable d'être administré au niveau du site de la lésion en utilisant un système d'administration par cathéter.

8. Produit médical selon l'une quelconque des revendications 1 à 7, dans lequel le produit médical comprend plus de 10 % en masse de l'agent muco-adhésif.

9. Produit médical selon l'une quelconque des revendications 1 à 8 pour utilisation dans le traitement d'une lésion gastro-intestinale.

10. Produit médical pour utilisation selon la revendication 9, dans lequel les lésions gastro-intestinales résultent d'une dissection sous-muqueuse endoscopique, d'une résection muqueuse endoscopique, d'une polypectomie, d'ulcères, de cancers, de varices, de l'ablation de l'endobrachyœsophage, d'une infection, d'anastomoses, de fistules ou d'une combinaison de ceux-ci.

11. Produit médical pour utilisation selon la revendication 9 ou la revendication 10, dans lequel le traitement comprend l'application ou l'administration du produit médical au niveau ou autour du site de la lésion par des techniques endoscopiques, des techniques laparoscopiques, ou de façon chirurgicale.

12. Produit médical pour utilisation selon la revendication 11, dans lequel le traitement comprend l'administration du produit médical en utilisant un système d'administration par cathéter.

13. Produit médical pour utilisation selon l'une quelconque des revendications 9 à 12, dans lequel le traitement comprend la pulvérisation, l'éjection ou l'étalement du produit médical au niveau et autour du site de la lésion.

14. Produit médical pour utilisation selon l'une quelconque des revendications 9 à 13, dans lequel le traitement comprend l'administration du produit médical en utilisant un système d'administration intraluminal.
